# EUROPEAN PATENT SPECIFICATION

(11) **EP 3 018 105 B1**
(45) Date of publication and mention of the grant of the patent: **19.06.2019**
(21) Application number: 13888563.7
(22) Date of filing: 12.12.2013
(51) Int. Cl.: C02F 3/30, C02F 11/04, C12P 7/62

(54) **ENHANCED SEWAGE BIOLOGICAL NITROGEN AND PHOSPHORUS REMOVAL METHOD BASED ON POLYHYDROXYALKANOATES METABOLIC REGULATION**
VERBESSERTES VERFAHREN ZUR ENTFERNUNG VON BIOLOGISCHEM STICKSTOFF UND PHOSPHOR AUS ABWASSERN AUF BASIS EINER STOFFWECHSELREGULIERUNG DURCH POLYHYDROXYALKANOATE
PROCÉDÉ BIOLOGIQUE AMÉLIORÉ D'ÉLIMINATION D'AZOTE ET DE PHOSPHORE D'EAUX USÉES BASÉ SUR LA RÉGULATION MÉTABOLIQUE DE POLYHYDROXYALCANOATES

(30) Priority: 03.07.2013 CN 201310278004
(43) Date of publication of application: 11.05.2016
(73) Proprietor: Tongji University, Shanghai 200092 (CN); Nanjing University, Nanjing, Jiangsu 210008 (CN)
(72) Inventor: CHEN, Yinguang, Shanghai 200092 (CN); ZHENG, Xiong, Shanghai 200092 (CN); REN, Hongqiang, Nanjing Jiangsu 210008 (CN); SU, Yinglong, Shanghai 200092 (CN); TANG, Shijing, Shanghai 200092 (CN)
(74) Representative: Wright, Howard Hugh Burnby
(86) International application number: PCT/CN2013/089203
(87) International publication number: WO 2015/000266

(56) References cited:
- CN-A- 101 070 217
- CN-A- 103 332 829
- JP-A- 2000 189 183
- US-A1- 2010 200 498
- TONG J ET AL: "Recovery of nitrogen and phosphorus from alkaline fermentation liquid of waste activated sludge and application of the fermentation liquid to promote biological municipal wastewater treatment", WATER RESEARCH, ELSEVIER, AMSTERDAM, NL, vol. 43, no. 12, 1 July 2009 (2009-07-01), pages 2969-2976, XP026170945, ISSN: 0043-1354, DOI: 10.1016/J.WATRES.2009.04.015 [retrieved on 2009-04-19]
- TONG, JUAN: 'Study on Short Chain Fatty Acid Generated by Suplus Sludge Alkaline Fermentation as A Carbon Source for Denitrification and Phosphorus Removal' CHINA DOCTORAL DISSERTATIONS FULL-TEXT DATABASE January 2008, pages 3 - 81, XP008181751
- ZHU, XIAOYU ET AL.: 'The Study On Short-Chain Fatty Acids Generated By Utilization Of Sewage Sludge' TECHNOLOGY OF WATER TREATMENT vol. 36, no. 10, October 2010, page 3, XP008181432
- HONG, CHEN ET AL.: 'Polyhydroxyalkanoate Production from Fermented Volatile Fatty Acids: Effect of Ph and Feeding Regimes.' BIORESOURCE TECHNOLOGY vol. 128, January 2013, page 534, XP055284689

## Description

### Technical Field

The present invention belongs to the technical field of biological treatment of water, wastewater or sewage, and relates to a method for enhanced biological removal of phosphorus and nitrogen from urban sewage through *in-situ* carbon source regulation of metabolism of polyhydroxyalkanoate (PHA).

### Background Art

With development of economy and improvement of living standards, the discharge of industrial wastewater and domestic sewage increases year by year, and the problem of water body pollution become increasingly serious. A large quantity of phosphorus, nitrogen and other nutrient substances flow into natural water bodies such as rivers, lakes and the like, resulting in widespread phenomenon regarding eutrophication. The discharge of sewage containing nitrogen and phosphorus is the main cause of eutrophication of water bodies. Activated sludge method, thanks to its advantages such as easy to operate, has become a major method for removal of nitrogen and phosphorus from sewage. However, on one hand, this method could lead to greenhouse gas emission, large amounts of primary and secondary sludge that are difficult to be treated and other problems; on the other hand, a common problem that the concentration of carbon source in urban sewage is relatively low also exists and it is very hard for sewage with a lower ratio of carbon to nitrogen to meet the increasingly elevated requirements for removal of phosphorus and nitrogen. Therefore, the sewage plants currently face multiple pressures such as up-to-standard discharge of nitrogen and phosphorus, sludge treatment, reduction in greenhouse gas emission and the like.

Studies show that in an activated sludge treatment system, microorganism is able to convert sewage organics into intracellular polyhydroxyalkanoate (PHA) under anaerobic or anoxic conditions, which mainly includes polyhydroxyvalerate (PHV) and polyhydroxybutyrate (PHB), wherein the denitrifying microorganisms can utilize the sewage organics or PHA as an electron donor for nitrate or nitrite under anoxic conditions to promote denitrification reaction; the microorganisms for removal of phosphorus are able to absorb phosphorus by utilizing the energy produced by oxidizing the intracellular PHA under an aerobic or anoxic environment. It can be seen that PHA is an important substance that has a direct effect on biological removal of phosphorus and nitrogen from sewage. If the metabolism of PHA can be regulated inside the sewage treatment plants to achieve an improved effect of phosphorus &nitrogen removal and a reduced greenhouse gas emission, it will provide a new technical support for upgrading and renovating the sewage plants as well as for their up-to-standard discharge, thereby realizing a sustainable development of the sewage plants.

CN 103332829 A discloses a method for biological removal of phosphorus from sewage comprising anaerobic fermentation of sludge whereby a treatment fluid is obtained which is directly added into municipal sewage, whereby polyhydroxyalkanoates are produced and phosphorus is absorbed.

### Summary of the Invention

The present invention aims to overcome the deficiencies of the prior art by providing a method for enhanced biological removal of phosphorus and nitrogen from sewage on the basis of metabolic regulation of polyhydroxyalkanoate.

To achieve above object, the present invention employs the following technical solutions:
The present invention newly adds bioconversion units in sewage treatment facilities, which *in situ* utilizes excess sludge and/or particulate organic substances produced from sewage treatment plants to make them efficiently and directionally convert into PHA precursors and then makes the PHA precursors efficiently convert into PHA, especially PHV, through process control method, which is necessary for removal of phosphorus and nitrogen, by which microorganisms in the sewage treatment system can be able to make use of PHA to fulfill a significant increase in efficiency of removal of phosphorus and nitrogen from sewage, thereby finally facilitating up-to-standard discharge of urban sewage.

The present invention discloses a new technique for enhanced removal of phosphorus and nitrogen by *in-situ* carbon source regulation of PHA metabolism (in-situ Carbon-Source Enhanced Technology, ICET). By way of regulation of metabolism of PHA, the efficiency of biological removal of phosphorus and nitrogen from sewage can be significantly increased. As a result, sewage effluent from the sewage treatment plants is capable of being discharged up-to-standard.

The present invention discloses a method for enhanced biological removal of phosphorus and nitrogen from sewage on the basis of metabolic regulation of polyhydroxyalkanoate, comprising the following steps:
(1), leading particulate organic substances obtained by primary sedimentation of the sewage and excess sludge discharged from a secondary sedimentation tank into a sludge thickening tank for condensation;
(2), placing the condensed organic wastes obtained from step (1) into a polyhydroxyalkanoate precursors (PHA precursors) production reactor to synthesize PHA precursors through anaerobic fermentation;
(3), after removing phosphorus and nitrogen from a mixture obtained by the anaerobic fermentation in a sludge conditioner, pumping the liquid into a PHA precursor reservoir;
(4), shortly afterwards, pumping the PHA precursors into a PHA production reactor to produce PHA;
(5), pumping the PHA obtained from step (4) into a conventional bioreactor for removal of phosphorus and nitrogen from sewage to conduct a process for removing phosphorus and nitrogen from sewage; wherein in step (1), the particulate organic substances obtained by primary sedimentation and the excess sludge discharged from the secondary sedimentation tank at a ratio between 1 : 10 to 10 : 1 calculated in the amount of volatile suspended solids (VSS) are introduced into the sludge thickening tank to condense for from 12 to 48 hours; wherein in step (2), pH value of the anaerobic fermentation ranges from 8.0 to 10.0 and the anaerobic fermentation lasts for from 8 to 10 days, during which the condensed organic wastes are directionally converted into the PHA precursors, wherein the PHA precursors mainly incorporate acetic acid and propionic acid at a percentage of from 30% to 60% and from 20% to 50%, respectively; and wherein in step (4), the polyhydroxyalkanoate precursors react at a pH value ranging from 6.8 to 7.2 for 3.5 hours to produce the polyhydroxyalkanoate (PHA).

In a preferred embodiment of the invention, in step (2), the raw materials for synthesis of the PHA precursors are selected from one or more than one of the sewage organics, the particulate organic substances in the primary sedimentation tank or the excess sludge in the secondary sedimentation tank.

In a further preferred embodiment of the invention, the bioreactor for biological removal of phosphorus and nitrogen from sewage used in step (5) is a sequencing batch reactor (SBR), an anaerobic/aerobic reactor (A/O), an anaerobic/anoxic/aerobic (A²/O) reactor, an anoxic/anaerobic/aerobic reactor (inverted A²/O) or an oxidation ditch.

Since dissolution and hydrolyzation of the sludge and the particulate organic substances (mainly protein and polysaccharide) in sewage can be accelerated under alkaline pH conditions and at the same time the activity of methane producing bacteria can be inhibited. As a result, the concentration of the PHA precursors produced at alkaline pH (such as pH value =10) is at least three times than those produced where pH = 7. The propionic acid content is able to reach 47% through regulation of ratio of carbon to nitrogen. The PHA precursors are converted into the PHA in the PHA production reactor, after which the organisms in activated sludge are able to utilize PHA as energy and carbon sources for their growth, denitrification and dephosphorization. Meanwhile PHV component in the PHA are internal carbon sources having a slow oxidization rate, which is capable of slowing down the rate at which carbon sources are directly oxidized by oxygen and then helps to enhance the denitrification and dephosphorization capabilities of the system.

The present invention discloses a new method for enhanced biological removal of phosphorus and nitrogen from sewage on the basis of metabolic regulation of polyhydroxyalkanoate and the recommended process parameters of the method are the following. The PHA precursors are obtained by anaerobic fermentation of the organic waste that is a mixture of substances from the primary and secondary sedimentation tanks at a pH value between 8.0 and 10.0 (the most preferable pH value=10). The PHA is obtained by the way in which the PHA precursors react for 3.5 hours at a pH value between 6.8 and 7.2 (the most preferable pH value=7).

The invention has the following advantages:
(1), the present method significantly raises the efficiency with which organic substances in sewage and sludge are directionally converted in order to synthesize PHA precursors and the propionic acid content in short chain fatty acid (SCFA), enabling the level of SCFA production at room temperature to increase by more than three times and the propionic acid content to increase from 20% to 47% or above while making sludge production reduce more than 20%.
(2), the present method significantly raises the efficiency with which PHA precursors are directionally converted into PHA, enabling the synthetic amount of PHA to account for around 70% of sludge in dry weight, which is apparently higher than that synthesized at a pH value between 7.6 and 8.0 (around 42%), and the PHV content in the synthesized PHA reaches up to about 65%.
(3), the PHA synthesized by the present method is easy to be used by the microorganisms in the sewage treatment system so that the efficiency of removal of phosphorus and nitrogen from sewage increases to around 80% to 95%, and the effluent water reaches the requirements set up by the national Grade 1A discharge standard.
(4), the method has easy to control processes and low cost of technical renovation, which is conducive to a large scale promotion.

### Brief Description of the Drawings

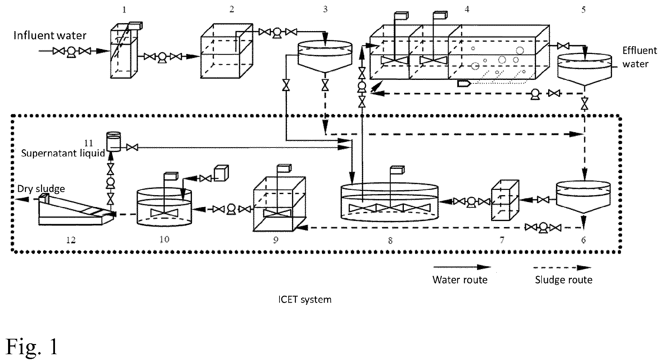

Fig. 1 is a process flow diagram of a new technique for enhanced removal of phosphorus and nitrogen through *in-situ* carbon source regulation of PHA metabolism (In-situ Carbon-Source Enhanced Technology, ICET).

**Numbers in the drawing:**

| | | | |
|---|---|---|---|
| 1 | Grid, | 2 | Regulation pool, |
| 3 | Primary sedimentation tank, | | |
| 4 | bioreactor for removal of phosphorus and nitrogen from sewage, | | |
| 5 | Secondary sedimentation tank, | 6 | Sludge thickening tank, |
| 7 | Supernatant liquid reservoir, | 8 | PHA production reactor, |
| 9 | PHA precursor production reactor, | 10 | Sludge conditioner, |
| 11 | PHA precursor reservoir, | 12 | Sludge dehydrator. |

### Detailed Description of the Preferred Embodiments

The following is a further explanation of the present invention in combination with the examples and figure.

### Example 1

(1) As shown in Fig. 1, introducing the organic wastes obtained from sewage which flows through a grid 1, a regulation pool 2 and a primary sedimentation tank 3and the excess sludge from a secondary sedimentation tank 5 into a sludge thickening tank 6 at a ratio of 1 : 1 calculated in VSS amount for condensation and precipitation for 24 hours;
(2) placing the condensed mixture into a PHA precursor production reactor 9 (anaerobic fermentation is carried out for 8 days at a pH value equal to 10.0) to make it directionally convert into PHA precursors;
(3) leading the obtained PHA precursors (at a concentration of 4650 mg COD/L and acetic acid and propionic acid content is 48% and 41% , respectively) into a sludge conditioner 10 for removal of phosphorus and nitrogen and then into a sludge dehydrator 12, and pumping the obtained liquid into a PHA precursor reservoir 11 for storage;
(4) pumping the liquid in a supernatant liquid reservoir 7 and the PHA precursors into a PHA production reactor 8 (reaction lasts for 3.5 hours at a pH value equal to 7.0) to synthesize PHA;
(5) pumping the activated sludge containing the PHA (synthetic amount of PHA reaches 2.42 mmol-C/g-VSS and PHV accounts for 65% of the PHA) into a A²/O bioreactor for removal of phosphorus and nitrogen from sewage 4. The actual chemical oxygen demand (COD) of the sewage to be treated is 150 mg/L, total nitrogen (TN) is 30.5 mg/L, total phosphorus (TP) is 6.0 mg/L, mixed liquor suspended solids (MLSS) is around 3100 mg/L, and sludge retention time (SRT) is about 20 d. The computational results are the following: COD = 45 mg/L; TN =7.0 mg/L; TP=0.4 mg/L; and NH₄⁺-N = 0 mg/L. The quality of effluent water meets the requirement set up by the Grade 1A discharge standard for sewage treatment plants.

### Example 2

(1) As shown in Fig. 1, introducing the organic wastes obtained from sewage which flows through a grid 1, a regulation pool 2 and a primary sedimentation tank 3and the excess sludge from a secondary sedimentation tank 5 into a sludge thickening tank 6 at a ratio of 10 : 1 calculated in VSS amount for condensation and precipitation for 12 hours;
(2) placing the condensed mixture into a PHA precursor production reactor 9 (anaerobic fermentation is carried out for 8 days at a pH value equal to 8.0);
(3) leading the obtained PHA precursors (at a concentration of 4230 mg COD/L and acetic acid and propionic acid content is 56% and 28% , respectively) into a sludge conditioner 10 for removal of phosphorus and nitrogen and then into a sludge dehydrator 12, and pumping the obtained liquid into a PHA precursor reservoir 11 for storage;
(4) pumping the liquid in a supernatant liquid reservoir 7 and the PHA precursors into a PHA production reactor 8 (reaction lasts for 3.5 hours at a pH value equal to 6.8) to synthesize PHA;
(5) pumping the activated sludge containing the PHA (synthetic amount of PHA reaches 2.33 mmol-C/g-VSS and PHV accounts for 61% of the PHA) into a A²/O bioreactor for removal of phosphorus and nitrogen from sewage 4. The actual chemical oxygen demand (COD) of the sewage to be treated is 130 mg/L, total nitrogen (TN) is 35mg/L, total phosphorus (TP) is 6.5 mg/L, mixed liquor suspended solids (MLSS) is around 3200 mg/L, and sludge retention time (SRT) is about 20 d. The computational results are the following: COD = 48 mg/L; TN =6.5 mg/L; TP=0.2 mg/L; and NH₄⁺-N = 0 mg/L. The quality of effluent water meets the requirement set up by the Grade 1A discharge standard for sewage treatment plants.

### Example 3

(1) As shown in Fig. 1, introducing the organic wastes obtained from sewage which flows through a grid 1, a regulation pool 2 and a primary sedimentation tank 3 and the excess sludge from a secondary sedimentation tank 5 into a sludge thickening tank 6 at a ratio of 5 : 1 calculated in VSS amount for condensation and precipitation for 24 hours;
(2) placing the condensed mixture into a PHA precursor production reactor 9 (anaerobic fermentation is carried out for 8 days at a pH value equal to 8.0);
(3) leading the obtained PHA precursors (at a concentration of 4310 mg COD/L and acetic acid and propionic acid content is 50% and 31% , respectively) into a sludge conditioner 10 for removal of phosphorus and nitrogen and then into a sludge dehydrator 12, and pumping the obtained liquid into a PHA precursor reservoir 11 for storage;
(4) pumping the liquid in a supernatant liquid reservoir 7 and the PHA precursors into a PHA production reactor 8 (reaction lasts for 3.5 hours at a pH value equal to 7.2) to synthesize PHA;
(5) pumping the activated sludge containing the PHA (synthetic amount of PHA reaches 2.35 mmol-C/g-VSS and PHV accounts for 62% of the PHA) into an inverted A²/O bioreactor for removal of phosphorus and nitrogen from sewage 4. The actual chemical oxygen demand (COD) of the sewage to be treated is 180 mg/L, total nitrogen (TN) is 30mg/L, total phosphorus (TP) is 5.5 mg/L, mixed liquor suspended solids (MLSS) is around 3000 mg/L, and sludge retention time (SRT) is about 20 d. The computational results are the following: COD = 42 mg/L; TN =7.5 mg/L; TP=0.1 mg/L; and NH₄⁺-N = 0.2 mg/L. The quality of effluent water meets the requirement set up by the Grade 1A discharge standard for sewage treatment plants.

### Example 4

(1) As shown in Fig. 1, introducing the organic wastes obtained from sewage which flows through a grid 1, a regulation pool 2 and a primary sedimentation tank 3 and the excess sludge from a secondary sedimentation tank 5 into a sludge thickening tank 6 at a ratio of 2 : 1 calculated in VSS amount for condensation and precipitation for 24 hours;
(2) placing the condensed mixture into a PHA precursor production reactor 9 (anaerobic fermentation is carried out for 8 days at a pH value equal to 9.0);
(3) leading the obtained PHA precursors (at a concentration of 4480 mg COD/L and acetic acid and propionic acid content is 46% and 38% , respectively) into a sludge conditioner 10 for removal of phosphorus and nitrogen and then into a sludge dehydrator 12, and pumping the obtained liquid into a PHA precursor reservoir 11 for storage;
(4) pumping the liquid in a supernatant liquid reservoir 7 and the PHA precursors into a PHA production reactor 8 (reaction lasts for 3.5 hours at a pH value equal to 6.8) to synthesize PHA;
(5) pumping the activated sludge containing the PHA (synthetic amount of PHA reaches 2.42mmol-C/g-VSS and PHV accounts for 64% of the PHA) into an inverted A²/O bioreactor for removal of phosphorus and nitrogen from sewage 4. The actual chemical oxygen demand (COD) of the sewage to be treated is 120 mg/L, total nitrogen (TN) is 31.5mg/L, total phosphorus (TP) is 7.3 mg/L, mixed liquor suspended solids (MLSS) is around 3200 mg/L, and sludge retention time (SRT) is about 20 d. The computational results are the following: COD = 40 mg/L; TN =6.3 mg/L; TP=0.2 mg/L; and NH₄⁺-N = 0.1 mg/L. The quality of effluent water meets the requirement set up by the Grade 1A discharge standard for sewage treatment plants.

### Example 5

(1) As shown in Fig. 1, introducing the organic wastes obtained from sewage which flows through a grid 1, a regulation pool 2 and a primary sedimentation tank 3 and the excess sludge from a secondary sedimentation tank 5 into a sludge thickening tank 6 at a ratio of 1 : 10 calculated in VSS amount for condensation and precipitation for 48 hours;
(2) placing the condensed mixture into a PHA precursor production reactor 9 (anaerobic fermentation is carried out for 8 days at a pH value equal to 10.0);
(3) leading the obtained PHA precursors (at a concentration of 4570 mg COD/L and acetic acid and propionic acid content is 55% and 30%, respectively) into a sludge conditioner 10 for removal of phosphorus and nitrogen and then into a sludge dehydrator 12, and pumping the obtained liquid into a PHA precursor reservoir 11 for storage;
(4) pumping the liquid in a supernatant liquid reservoir 7 and the PHA precursors into a PHA production reactor 8 (reaction lasts for 3.5 hours at a pH value equal to 7.2) to synthesize PHA;
(5) pumping the activated sludge containing the PHA (synthetic amount of PHA reaches 2.39 mmol-C/g-VSS and PHV accounts for 62% of the PHA) into a SBR bioreactor for removal of phosphorus and nitrogen from sewage 4. The actual chemical oxygen demand (COD) of the sewage to be treated is 160 mg/L, total nitrogen (TN) is 29 mg/L, total phosphorus (TP) is 8.6 mg/L, mixed liquor suspended solids (MLSS) is around 3100 mg/L, and sludge retention time (SRT) is about 20 d. The computational results are the following: COD = 39 mg/L; TN =6.5 mg/L; TP=0.3 mg/L; and NH₄⁺-N = 0.2 mg/L. The quality of effluent water meets the requirement set up by the Grade 1A discharge standard for sewage treatment plants.

The above descriptions of examples are conducive for ordinary technicians of the present technical field to understand and exploit the invention. It is obvious that persons skilled in the art of the present field can easily make various amendments to the above examples and apply the general principle illustrated in here into other examples without the effort of inventive work. Therefore, the present invention is not confined to examples herein. Any improvements and modifications conducted by persons skilled in the art of the present field according to the instructions of the present invention and without going beyond the scope of the present invention shall be included in the extent of protection of the present invention.

## Claims

1. A method for enhanced biological removal of phosphorus and nitrogen from sewage on the basis of metabolic regulation of polyhydroxyalkanoate, comprising the following steps:
(1), leading particulate organic substances obtained by primary sedimentation of the sewage and excess sludge discharged from a secondary sedimentation tank into a sludge thickening tank for condensation;
(2), placing the condensed organic wastes obtained from step (1) into a polyhydroxyalkanoate precursor production reactor to synthesize polyhydroxyalkanoate precursors through anaerobic fermentation;
(3), after removing phosphorus and nitrogen from a mixture obtained by the anaerobic fermentation in a sludge conditioner, pumping the liquid into a polyhydroxyalkanoate precursor reservoir;
(4), shortly afterwards, pumping the polyhydroxyalkanoate precursors into a polyhydroxyalkanoate production reactor to produce polyhydroxyalkanoate;
(5), pumping the polyhydroxyalkanoate obtained from step (4) into a conventional bioreactor for biological removal of phosphorus and nitrogen from sewage to conduct a process for removing phosphorus and nitrogen from sewage;
wherein in step (1), the particulate organic substances obtained by primary sedimentation and the excess sludge discharged from the secondary sedimentation tank at a ratio between 1 : 10 and 10 : 1 by the amount of volatile suspended solids are introduced into the sludge thickening tank to condense for from 12 to 48 hours;
wherein in step (2), pH value of the anaerobic fermentation ranges from 8.0 to 10.0 and the anaerobic fermentation lasts for from 8 to 10 days, during which the condensed organic wastes are directionally converted into the polyhydroxyalkanoate precursors, wherein the polyhydroxyalkanoate precursors incorporate acetic acid and propionic acid at a percentage of from 30% to 60% and from 20% to 50%, respectively;
wherein in step (4), the polyhydroxyalkanoate precursors reacts at a pH value ranging from 6.8 to 7.2 for 3.5 hours to produce the polyhydroxyalkanoate.

2. The method according to claim 1, wherein in step (2), the raw materials for synthesis of the polyhydroxyalkanoate precursors are selected from one or more than one of the sewage organics, the particulate organic substances in the primary sedimentation tank or the excess sludge in the secondary sedimentation tank.

3. The method according to claim 1, wherein the bioreactor for biological removal of phosphorus and nitrogen from sewage used in step (5) is a sequencing batch reactor, an anaerobic/aerobic reactor, an anaerobic/anoxic/aerobic reactor, an anoxic/anaerobic/aerobic reactor or an oxidation ditch.

## Patentansprüche

1. Verfahren zur verstärkten biologischen Phosphor- und Stickstoffentfernung aus Abwasser, basierend auf der Stoffwechselregulierung von Polyhydroxyalkanoat, die folgenden Schritte umfassend:
(1) Leiten organischer Schwebstoffe, die durch Vorklären von Abwasser erhalten wurden, und Überschussschlammes aus einem sekundären Absetzbecken in ein Schlammeindickbecken zum Eindicken,
(2) Bringen des eingedickten organischen Abfalles, der in Schritt (1) erhalten wurde, in einen Reaktor zur Erzeugung von Polyhydroxyalkanoat-Vorläufern zur Synthese von Polyhydroxyalkanoat-Vorläufern durch anaerobe Gärung,
(3) nach Entfernen von Phosphor und Stickstoff aus einem Gemisch, dass durch anaerobe Gärung in einem Klärschlammaufbereiter erhalten wurde, Pumpen der Flüssigkeit in einen Behälter für Polyhydroxyalkanoat-Vorläufer,
(4) kurz danach Pumpen der Polyhydroxyalkanoat-Vorläufer in einen Reaktor zur Erzeugung von Polyhydroxyalkanoat, um Polyhydroxyalkanoat zu erzeugen,
(5) Pumpen des in Schritt (4) erhaltenen Polyhydroxyalkanoats in einen herkömmlichen Bioreaktor für biologische Phosphor- und Stickstoffentfernung aus Abwasser zur Ausführung eines Vorganges zur Phosphor- und Stickstoffentfernung aus Klärschlamm,
wobei in Schritt (1) die organischen Schwebstoffe, die durch Vorklären erhalten wurden, und der Überschussschlamm aus dem sekundären Absetzbecken in einem Verhältnis zwischen 1:10 und 10:1 der Menge flüchtiger Schwebstoffe für 12 bis 48 Stunden zum Eindicken in ein Schlammeindickbecken gebracht werden,
wobei in Schritt (2) der pH-Wert der anaeroben Gärung zwischen 8,0 und 10,0 liegt und die anaerobe Gärung während 8 bis 10 Tagen erfolgt, während welcher Zeit die eingedickten organischen Abfälle gerichtet in die Polyhydroxyalkanoat-Vorläufer umgesetzt werden, wobei die Polyhydroxyalkanoat-Vorläufer Essigsäure und Propionsäure in einem Prozentsatz von 30% bis 60% bzw. von 20% bis 50% enthalten,
wobei in Schritt (4) die Polyhydroxyalkanoat-Vorläufer bei einem pH-Wert zwischen 6,8 und 7,2 für 3,5 Stunden reagieren, um das Polyhydroxyalkanoat zu erzeugen.

2. Verfahren nach Patentanspruch 1, wobei in Schritt (2) das Rohmaterial zur Synthese der Polyhydroxyalkanoat-Vorläufer unter einem oder mehreren unter organischen Abwassersubstanzen, organischen Schwebstoffen im Vorklärbecken und Überschussschlamm aus dem sekundären Absetzbecken gewählt werden.

3. Verfahren nach Patentanspruch 1, wobei der Bioreaktor für biologische Phosphor- und Stickstoffentfernung aus Abwasser, der in Schritt (5) verwendet wird, ein SBR-Reaktor (Sequencing-Batch-Reactor) ist, ein anaerob-aerober Reaktor, ein anaerob-anoxischaerober Reaktor, ein anoxisch-anaerob-aerober Reaktor oder ein Oxidationsgraben.

## Revendications

1. Procédé permettant une élimination biologique améliorée du phosphore et de l'azote dans des eaux usées basé sur la régulation métabolique des polyhydroxyalcanoates, comprenant les étapes suivantes consistant à :
(1) transférer des substances organiques particulaires obtenues par sédimentation primaire des eaux usées et des boues en excès évacuées d'une cuve de sédimentation secondaire dans une cuve d'épaississement des boues pour permettre leur condensation,
(2) introduire les produits résiduaires organiques condensés obtenus à l'issue de l'étape (1) dans un réacteur de production de précurseurs de polyhydroxyalcanoates pour synthétiser des précurseurs de polyhydroxyalcanoates par fermentation anaérobie,
(3) après avoir éliminé le phosphore et l'azote du mélange obtenu par la fermentation anaérobie dans un conditionneur de boues, pomper le liquide dans un réservoir de précurseurs de polyhydroxyalcanoates,
(4) peu après, pomper les précurseurs de polyhydroxyalcanoates dans un réacteur de production de polyhydroxyalcanoates pour produire des polyhydroxyalcanoates,
(5) pomper les polyhydroxyalcanoates obtenus à l'issue de l'étape (4) dans un bioréacteur conventionnel pour permettre l'élimination biologique du phosphore et de l'azote des eaux usées de façon à mettre en oeuvre un procédé d'élimination du phosphore et de l'azote des eaux usées,
lors de l'étape (1), les substances organiques particulaires obtenues par sédimentation primaire et les boues en excès évacuées de la cuve de sédimentation secondaire étant introduites selon un rapport compris entre 1 :10 et 10 :1, en quantité de solides volatiles en suspension dans la cuve d'épaississement des boues pour permettre leur condensation pendant 12 à 48 heures,
lors de l'étape (2) la valeur du pH de la fermentation anaérobie étant située dans la plage comprise entre 8,0 et 10,0 et la fermentation anaérobie durant entre 8 et 10 jours, au cours desquels les produits résiduaires organiques condensés sont transformés de manière directionnelle en précurseurs de polyhydroxyalcanoates, les précurseurs de polyhydroxyalcanoates renfermant de l'acide acétique et de l'acide propionique, respectivement, selon un pourcentage compris entre 30% et 60% et entre 20% et 50%,
lors de l'étape (4), les précurseurs de polyhydroxyalcanoates réagissant à une valeur de pH située dans la plage de 6,8 à 7,2 pendant 3,5 heures pour produire les polyhydroxyalcanoates.

2. Procédé conforme à la revendication 1,
selon lequel lors de l'étape (2) les matériaux bruts pour la synthèse des précurseurs de polyhydroxyalcanoates sont des composés organiques des eaux usées, et/ou des substances organiques particulaires renfermées dans la cuve de sédimentation primaire et/ou les boues en excès renfermées dans la cuve de sédimentation secondaire.

3. Procédé conforme à la revendication 1,
selon lequel le bioréacteur destiné à l'élimination biologique du phosphore et de l'azote des eaux usées utilisé lors de l'étape (5) est un réacteur à bâchées discontinues, un réacteur anaérobie/aérobie, un réacteur anaérobie/anoxique/aérobie, un réacteur anoxique/anaérobie/aérobie ou un fossé d'oxydation.
